(19) Europäisches Patentamt
European Patent Office
Office européen des brevets

(11) Veröffentlichungsnummer: **0 232 675 B1**

# (12) EUROPÄISCHE PATENTSCHRIFT

(45) Veröffentlichungstag der Patentschrift: **22.11.90**

(51) Int. Cl.[5]: **C07D 471/04**, A61K 31/44 // (C07D471/04, 221:00, 209:00)

(21) Anmeldenummer: **86730212.7**

(22) Anmeldetag: **19.12.86**

(54) **5-Aminoalkyl-Beta-Carbolinderivate, ihre Herstellung und Verwendung als Arzneimittel.**

(30) Priorität: **20.12.85 DE 3545776**

(43) Veröffentlichungstag der Anmeldung: **19.08.87 Patentblatt 87/34**

(45) Bekanntmachung des Hinweises auf die Patenterteilung: **22.11.90 Patentblatt 90/47**

(84) Benannte Vertragsstaaten: **AT BE CH DE ES FR GB GR IT LI LU NL SE**

(56) Entgegenhaltungen:
**EP-A- 0 030 254**
**EP-A- 0 130 140**
**EP-A- 0 130 141**
**EP-A- 0 137 390**
**EP-A- 0 161 574**

**PATENT ABSTRACTS OF JAPAN, unexamined applications, Field C, Vol. 6,No. 63, 22. April 1982 THE PATENT OFFICE JAPANESE GOVERNMENT Seite 138 C 99**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen, Müllerstrasse 170/178 Postfach 65 03 11, D-1000 Berlin 65(DE)**

(72) Erfinder: **Biere, Helmut, Dr., Zeltinger Strasse 15, D-1000 Berlin 28(DE)**
Erfinder: **Huth, Andreas, Dr., Kirchweg 55, D-1000 Berlin 38(DE)**
Erfinder: **Rahtz, Dieter, Dr., Krottnaurerstrasse 24a, D-1000 Berlin 38(DE)**
Erfinder: **Schmiechen, Ralph, Dr., Bayernring 27, D-1000 Berlin 42(DE)**
Erfinder: **Seidelmann, Dieter, Dr., Stierstrasse 14, D-1000 Berlin 41(DE)**
Erfinder: **Stephens, David Norman, Dr., Hildegardstrasse 16A, D-1000 Berlin 31(DE)**

**EP 0 232 675 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft neue 5-Aminoalkyl-β-carbolinderivate, ihre Herstellung und ihre Verwendung als Arzneimittel.

Die erfindungsgemäßen Verbindungen besitzen wertvolle pharmakologische Eigenschaften. Sie beeinflußen insbesondere das Zentralnervensystem und eignen sich somit als Psychopharmaka.

Aus den EP-A 30 254 sind β-Carboline bekant, die im A-Ring mit einer Aminogruppe substituiert sind; diese β-Carboline zeigen aber geringe Affinität an die Benzodiazepin-Rezeptoren.

Die erfindungsgemäßen Verbindungen haben die allgemeine Formel I

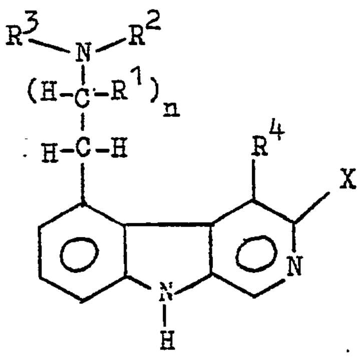

(I),

worin
n = 0 oder 1 ist,
$R^1$ Wasserstoff oder niederes Alkyl bedeutet,
$R^2$ und $R^3$ jeweils Wasserstoff, gegebenenfalls substituiertes niederes Alkyl, Acyl oder Aryl darstellen oder gemeinsam mit dem Stickstoffatom einen 5 - 6 gliedrigen Heterocyclus bilden,
$R^4$ Wasserstoff, niederes Alkyl oder niederes Alkoxyalkyl bedeutet und
X = einen Oxadiazolylrest der Formel

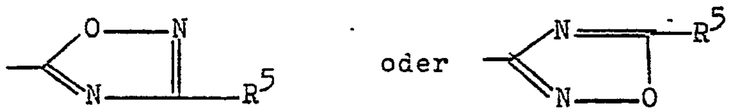

mit $R^5$ in der Bedeutung von H, niederem Alkyl oder Cycloalkyl darstellt
oder
eine $COOR^6$-Gruppe mit $R^6$ in der Bedeutung von H oder niederem Alkyl ist
oder eine $CO\text{-}NR^7R^8$-Gruppe mit $R^7$ und $R^8$ jeweils in der Bedeutung von Wasserstoff oder gegebenenfalls substituiertem niederem Alkyl, Acyl oder Aryl, wobei $R^7$ und $R^8$ gemeinsam mit dem Stickstoffatom einen 5 - 6 gliedrigen Heterocyclus bilden können.

Unter niederem Alkyl sind sowohl gerad- als auch verzweigtkettige Reste mit $C_1$-$C_6$ Kohlenstoffatomen zu verstehen. Beispielsweise seien die bevorzugten $C_{1-4}$-Alkylreste gennant wie Methyl, Ethyl, n-Propyl, iso-Propyl, n-Butyl, iso-Butyl und tert.-Butyl.

Als Substituenten der niederen Alkylreste $R^2$, $R^3$, $R^7$ und $R^8$ sind geeignet: Hydroxy, nieder-Alkoxy, Mercapto, nieder-Alkylthio, Phenyl, eine gegebenenfalls mit niederem Alkyl substituierte Aminogruppe oder ein stickstoffhaltiger 5 - 6 gliedriger Heterocyclus, der noch ein weiteres Heteroatom wie Schwefel, Stickstoff oder Sauerstoff enthalten kann wie Morpholin, Piperidin, Thiomorpholin, Piperazin und Pyrrolidin und mit 1 oder 2 niederen Alkylgruppen substituiert sein kann. Im Piperazinrest kann der Stickstoff in 4-Stellung zusätzlich durch einen niederen Alkylrest substituiert sein.

Bilden $R^2$ und $R^3$ oder $R^7$ und $R^8$ gemeinsam mit dem Stickstoffatom einen Heterocyclus, so ist dieser 5 - 6 gliedrig und kann gesättigt oder ungesättigt sein und ein weiteres Heteroatom wie Schwefel, Stickstoff oder Sauerstoff enthalten.

Beispielsweise sind die oben genannten gesättigten Heterocyclen wie auch die nachfolgend aufgeführten ungesättigten Heterocyclen geeignet : Imidazol, Pyrazol, Pyrrol.

Der Acylrest leitet sich bevorzugt von aliphatischen Carbonsäuren mit bis zu 4 Kohlenstoffatomen ab wie beispielsweise Essigsäure, Propionsäure, Buttersäure, Ameisensäure u. a.

Unter Aryl ist der Phenylrest und 5-6 gliedrige heteroaromatische Reste wie beispielsweise Furan, Thiophen, Pyridin zu verstehen.

Als bevorzugt für die Reste $R^7$ und $R^8$ seien $C_{1-3}$ Alkyle und gemeinsam mit dem Stickstoffatom 5-6 gliedrige stickstoffhaltige gesättigte Heterocyclen, die ein weiteres Heteroatom enthalten können genannt.

Der Cycloalkylrest $R^5$ kann 3-7 Kohlenstoffatome enthalten, wobei als bevorzugt Reste mit 3-5 Kohlenstoffatomen genannt seien wie beispielsweise Cyclopropyl, Methylcyclopropyl, Cyclobutyl, Cyclopentyl.

Es ist bekannt, daß bestimmte Stellen im zentralen Nervensystem von Vertebraten eine hohe spezifische Affinität für die Bindung von 1.4-und 1.5-Benzodiazepinen aufweisen (Squires, R. F. und Braestrup, C., Nature (London) 266 (1977), 734). Diese Stellen werden Benzodiazepin-Rezeptoren genannt.

Die für die pharmakologischen Eigenschaften der erfindungsgemäßen Verbindungen wichtige Rezeptoraffinität wurde durch Untersuchung des Verdrängungsvermögens von radioaktiv-markiertem Flunitrazepam von Benzodiazepin-Rezeptoren bestimmt.

Die Verdrängungsaktivität der erfindungsgemäßen Verbindungen wird als $IC_{50}$ -und $ED^{50}$-Wert angegeben. Der $IC^{59}$-Wert gibt die Konzentration an, die eine 50 %ige Verdrängung der spezifischen Bindung von $H^3$-Flunitrazepam (1,0 nM, 0°C) in Proben mit einem Gesamtvolumen von 0,55 ml einer Suspension von Hirnmembranen, z.B. von Ratten bewirkt.

Der Verdrängungstest wird wie folgt ausgeführt:

0,5 ml einer Suspension von unbehandeltem Rattenvorderhirn in 25 mM $KH_2PO_4$, pH = 7,1 (5-10 mg Gewebe/Probe) wird für 40 bis 60 Minuten bei 0°C zusammen mit $^3H$-Diazepam (spezifische Aktivität 14,4 Ci/mmol, 1,9 nM) oder $^3H$-Flunitrazepam (spezifische Aktivität 87 Ci/mmol, 1,0 nM) inkubiert. Nach Inkubation wird die Suspension durch eine Glasfritte filtriert, der Rückstand zweimal mit kalter Pufferlösung gewaschen und die Radioaktivität im Scintillationszähler gemessen.

Der Versuch wird dann wiederholt, jedoch so, daß vor der Zugabe des radioaktiv-markiertem Benzodiazepins eine bestimmte Menge oder eine überschüssige Menge der Verbindung, deren Verdrängungsaktivität zu bestimmen ist, zugesetzt wird. Auf Grundlage der erhaltenen Werte kann dann der $IC_{50}$-Wert berechnet werden.

Der $ED_{50}$-Wert stellt die Dosis einer Versuchssubstanz dar, die eine Reduktion der spezifischen Bindung des Flunitrazepams an dem Benzodiazepin-Rezeptor in einem lebenden Gehirn auf 50% des Konrollwertes bewirkt.

Der in-vivo Test wird wie folgt ausgeführt:

Gruppen von Mäusen wird die Versuchssubstanz bei unterschiedlichen Dosen und normalerweise intraperitoneal injuziert. Nach 15 Minuten wird den Mäusen das $^3H$-Flunitrazepam intravenös verabreicht. Nach weiteren 20 Minuten werden die Mäuse getötet, ihr Vorderhirn entfernt und die an die Hirnmembranen spezifisch gebundene Radioaktivität durch Scintillationszählung gemessen. Der $ED_{50}$-Wert wird aus den Dosis/Wirkungs-Kurven bestimmt.

Die erfindungsgemäßen Verbindungen zeigen im pharmakologischen Test insbesondere anxiolytische und antikonvulsive Wirksamkeit. Zur Untersuchung der antikonvulsiven Wirkung wird die Aufhebung der mit Pentylentetrazol (Pentazol) induzierten Krämpfe untersucht. Pentazol wird in einer Menge von 150 mg/kg als Salzsäure-Lösung (ph 2-3) subcutan 15-30 Minuten nach der intraperitonealen Applikation der Testsubstanz gegeben. Diese Menge induziert klonische und tonische Krämpfe, die bei unbehandelten Tieren zum Tode führen. Die Zahl der Mäuse, die Krämpfe zeigen und die Zahl derer, die 30 Minuten nach Pentazol gestorben sind, wird registriert.

Nach der Methode von Litchfield und Wilcoxon ( J.Pharmacol. exp. Ther.96 (1949) 99-103 werden die $ED_{50}$ - Werte als die Menge der antagonistisch wirkenden Substanz bestimmt, die 50 % der Tiere vor Krämpfen und Tod schützt.

Die neuen Verbindungen der allgemeinen Formel I besitzen wertvolle pharmakologische Eigenschaften. Insbesondere wirken sie sich auf das Zentralnervensystem aus und sind somit als Psychopharmaka für Humanmedizin geeignet. Die Verbindungen können besonders zur Behandlung von Angst begleitet von Depressionen, Epilepsie, Schlafstörungen, Spastizitäten und Muskelrelaxation während der Anästhesie eingesetzt werden. Die erfindungsgemäßen Verbindungen zeigen auch amnestische bzw. gedächtnisfördernde Eigenschaften.

Die erfindungsgemäßen Verbindungen können zur Formulierung von pharmazeutischen Präparationen, zum Beispiel für die orale und parenterale Anwendung nach an sich bekannten Methoden der Galenik verwendet werden.

Als Hilfsstoffe zur Formulierung von pharmazeutischen Präparationen sind solche physiologisch verträglichen organischen und anorganischen Trägersubstanzen für die enterale und parenterale Anwendung geeignet, die gegenüber den erfindungsgemäßen Verbindungen inert sind.

Als Trägersubstanzen seien beispielsweise genannt Wasser, Salzlösungen, Alkohole, Polyäthylenglykole, polyhydroxyethoxyliertes Rizinusöl, Gelatine, Lactose, Amylose, Magnesiumstearat, Talkum, Kieselsäure, Fettsäuremono- und diglyceride, Pentaerythritolfettsäureester, Hydroxymethylcellulose und Polyvinylpyrrolidon.

Die pharmazeutischen Präparationen können sterilisiert und/oder mit Hilfsstoffen, wie Schmiermitteln, Konservierungsstoffen, Stabilisatoren, Netzmitteln, Emulgatoren, Puffermitteln und Farbstoffen, versetzt werden.

Für die parenterale Anwendung sind insbesondere Injektionslösungen oder Suspensionen, insbesondere wässrige Lösungen der aktiven Verbindungen in Polyhydroxyethoxyliertem Rizinusöl, geeignet.

Für die orale Anwendung sind insbesondere Tabletten, Dragees oder Kapseln mit Talkum und/oder einem Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt wird.

Die erfindungsgemäßen Verbindungen werden in einer Dosiseinheit von 0,05 bis 100 mg aktiver Substanz in einem physiologisch verträglichen Träger eingebracht.

Die erfindungsgemäßen Verbindungen werden in einer Dosis von 0,1 bis 300 mg/Tag, vorzugsweise 1-30 mg/Tag, angewendet.

Die Herstellung der erfindungsgemäßen Verbindungen der allgemeinen Formel I erfolgt nach an sich bekannten Methoden.

Beispielsweise erfolgt die Herstellung der Verbindung der allgemeinen Formel I, dadurch, daß man

a) eine Verbindung der allgemeinen Formel II

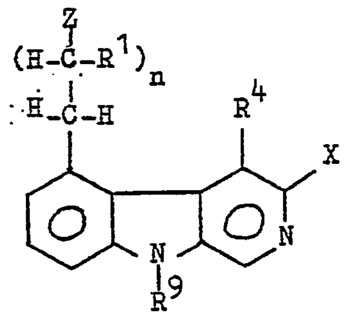

(II),

worin

$R^1$, $R^4$, X und n die oben angegebene Bedeutung haben,

Z Halogen oder Hydroxy ist und

$R^9$ Wasserstoff oder eine Schutzgruppe bedeutet

mit einer Verbindung $HNR^2R^3$, worin $R^2$ und $R^3$ die oben angegebene Bedeutung haben, umsetzt und anschließend gegebenenfalls die Schutzgruppe abspaltet,

b) eine Verbindung der allgemeinen Formel III

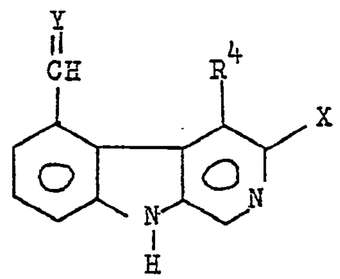

(III),

worin

$R^4$ und X die oben genannte Bedeutung haben und

Y eine $O_2N$-C-$R^1$- oder $R^2$N-Gruppe ist, worin $R^1$ die oben genannte Bedeutung hat und $R^2$ gegebenenfalls substituiertes niederes Alkyl oder Aryl darstellt,

hydriert zu einer Verbindung der allgemeinen Formel I mit $R^3$ in der Bedeutung von Wasserstoff und anschließend gegebenenfalls die nach Verfahren a) oder b) erhaltenen Verbindungen umestert oder die Ester verseift und gewünschtenfalls die so erhaltene Carbonsäure

α) amidiert

β) mit einer Verbindung

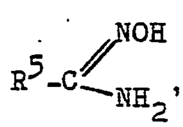

,

worin $R^5$ die oben genannte Bedeutung hat, umsetzt zu einer Verbindung der allgemeinen Formel I,in der X für den Rest

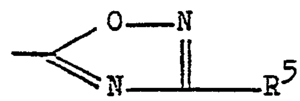

mit $R^5$ in der oben genannten Bedeutung steht,

c) eine Verbindung der allgemeinen Formel IV

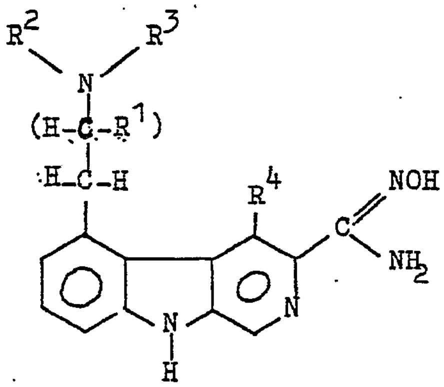

(IV),

worin

$R^1$, $R^2$, $R^3$, $R^4$ und n die oben genannte Bedeutung haben mit einem Carbonsäureanhydrid $(R^5CO)_2O$, worin $R^5$ die oben genannte Bedeutung hat, umsetzt zu einer Verbindung der allgemeinen Formel I, in der X für den Rest

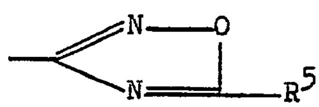

mit $R^5$ in der oben genannten Bedeutung steht.

Die Einführung der Aminogruppe nach Verfahren a) kann beispielsweise durch Umsetzung der entsprechenden Halogenverbindung mit primären oder sekundären Aminen erfolgen.

Als Halogen ist Chlor, Brom oder Jod geeignet. Als Lösungsmittel sind dipolar aprotische Lösungsmittel wie beispielsweise Dimethylsulfoxid, Dimethylformamid, N-Methylpyrrolidon u.a. oder protische Lösungsmittel wie beispielsweise Alkohole wie Methanol, Ethanol, Propanol u.a. oder chlorierte Kohlenwasserstoffe wie beispielsweise Chloroform, Methylenchlorid u.a. geeignet.

Ausgehend von Hydroxyalkylenverbindungen können nach den üblichen Methoden zum Beispiel über intermediär mit Phosphortrihalogenid hergestellte Halogenalkylenverbindungen in den oben genannten Lösungsmitteln gleich die entsprechenden Amine hergestellt werden.

Die Reaktionstemperatur geht von 0 °C bis zur Siedetemperatur des Lösungsmittels. Die Umsetzung ist im allgemeinen nach ca. 10 bis 24 Stunden beendet.

Ist in 9-Stellung eine übliche Schutzgruppe wie beispielsweise eine Acyl- oder Tosylschutzgruppe vorhanden, so wird diese bei der Umsetzung mit dem Amin abgespalten oder anschließend mit den üblichen Methoden entfernt beispielsweise durch Behandeln mit Basen wie Natrium- oder Kalium-carbonaten/hydroxiden oder -alkoholaten.

Die Aminierung kann mit oder ohne Inertgas wie beispielsweise Argon oder Stickstoff durchgeführt werden.

Werden im Verfahren a) β-Carbolin-3-carbonsäuren eingesetzt, so erhält man die entsprechenden β-Carbolin-3-carbonsäureamide.

Die Hydrierung der Verbindungen der allgemeinen Formel III nach Verfahren b) erfolgt vorzugsweise katalytisch zum Beispiel mit Edelmetallkatalysatoren wie Platin oder Palladium auf geeigneten Trägern wie Kohle oder mit Raney-Nickel.

Die Hydrierung wird bevorzugt in protischen Lösungsmitteln wie Alkoholen beispielsweise Ethanol, Methanol, Propanol u.a. bei Raumtemperatur bis zur Siedetemperatur des Lösungsmittels unter Normaldruck oder $H_2$-Druck vorgenommen.

Um Umesterungen zu vermeiden, arbeitet man in dem jeweiligen Alkohol der Esterkomponente als Lösungsmittel. Die Reaktion ist im allgemeinen nach 5 bis 7 Stunden beendet.

Nach Verfahren b) erhält man bei Hydrierung von Nitroverbindungen primäre Amine und bei Hydrierung von Iminen sekundäre Amine.

Wird eine Umesterung gewünscht, so kann man zum Beispiel mit dem entsprechenden Alkohol oder Alkalialkoholat umsetzen, gegebenenfalls kann man Titantetra-isopropylat als Katalysator im wasserfreiem Alkohol zufügen. Üblicherweise wird die Umesterung bei Temperaturen von 60 - 120 °C durchgeführt und ist nach ca. 2 - 6 Stunden beendet.

Die Einführung der tert.-Butylestergruppe erfolgt beispielsweise durch Umsetzung der Carbonsäure mit tert.-Butoxty-bis-(dimethylamino)methan. Im allgemeinen wird die Reaktion unter Inertgasatmosphäre wie Argon oder Stickstoff und unter Feuchtigkeitsausschluß bei erhöhter Temperatur durchgeführt.

Die Verseifung der Estergruppe kann sauer oder alkalisch erfolgen; vorzugsweise wird alkalisch verseift, indem der Ester mit verdünnter wässriger Alkalilauge wie Kalium- oder Natriumhydroxid, in einem protischen Lösungsmittel, wie zum Beispiel Methanol, Ethanol oder Ethylenglykol, auf Temperaturen bis zur Rückflußtemperatur des Reaktionsgemisches erhitzt wird.

Carbonsäureamide werden beispielsweise auch erhalten durch Umsetzung mit Aminen aus den entsprechenden Imidazoliden, die intermediär hergestellt werden aus den Carbonsäuren und Carbonyl- oder Thionyl- diimidazol. Die Reaktion wird bei Raumtemperatur in dipolaren aprotischen Lösungsmitteln durchgeführt wie beispielsweise Dimethylformamid, Dimethylacetamid u.a..

Zur Einführung des 1,2,4-Oxadiazol-5-yl-Restes wird die β-Carbolincarbonsäure mit einem Amidoxim der Formel

$R^5$-C(=NOH)$NH_2$

in einem inerten Lösungsmittel, das über 100 °C siedet und gegenüber dem Reaktanten inert ist, bei der Rückflußtemperatur des Reaktionsgemisches zur Kondensation gebracht. Geeignete Lösungsmittel für die Kondensationsreaktion sind beispielsweise Toluol und Dimethylformamid. Zweckmäßigerweise wird die freie β-Carbolin-3-carbonsäure vor der Kondensationsreaktion in geeigneter Weise aktiviert. Hierzu kann die freie Säure zum Beispiel in das gemischte Anhydrid. in den aktivierten Ester oder in das Chlorid überführt werden.

Gut bewährt hat sich auch eine Aktivierung zum Imidazolid mit Imidazol/Thionylchlorid oder auch Carbonyldiimidazol in einem aprotischen Lösungsmittel wie Dioxan, Tetrahydrofuran, Dimethylformamid oder N-Methylpyrrolidon bei Temperaturen zwischen 0 und 50 °C, vorzugsweise Raumtemperatur.

Zur Einführung des 1,2,4-Oxadiazol-3-yl-Restes setzt man beispielsweise das β-carbolin-3-carboxamidoxim der allgemeinen Formel IV bei Raumtemperatur mit dem Säureanhydrid $(R^5CO)_2O$ um und erwärmt anschließend bis zur Siedetemperatur. Die Reaktion ist nach ca. 7 Stunden beendet und wird nach dem üblichen Verfahren aufgearbeitet.

Die erfindungsgemäßen Verbindungen können als Racemat vorliegen oder werden nach den üblichen Verfahren in ihre Antiopoden aufgetrennt.

Die Herstellung der Ausgangsverbindungen ist bekannt oder erfolgt nach an sich bekannten Verfahren.

Beispielsweise werden 3-Carboxamidoxime aus den β-Carbolincarbonsäuren hergestellt, indem man das 3-Carbonsäurenitril mit Hydroxylamin umsetzt.

Die 5-Halogenmethyl-ausgangsverbindungen der allgemeinen Formel II sind beispielsweise durch Umsetzung der 5-Methylcarbolinverbindungen mit N-Halogensuccinimid insbesondere N-Brom-succinimid unter den üblichen Reaktionsbedingungen darstellbar.

Die nachfolgenden Beispiele sollen das erfindungsgemäße Verfahren erläutern.

Beispiel 1

5-Phenylaminomethyl-β-carbolin-3-carbonsäure-ethylester

5-Phenyliminomethyl-β-carbolin-3-carbonsäure-ethylester (0,25 g) werden in 100 ml Ethanol unter Zusatz von Raney-Nickel bei Normaldruck und einer Temperatur von 25 °C hydriert. Die Aufnahme von 1 Mol Wasserstoff nimmt etwa 40 Minuten in Anspruch. Der nach Abfiltrieren des Katalysators und Eindampfen des Filtrats verbleibende Rückstand wird an Kieselgel mit einem Gemisch aus 10 Teilen Dichlormethan und einem Teil Ethanol chromatographiert. So werden 0,15 g 5-Phenylaminomethyl-β-carbolin-3-carbonsäure-ethylester erhalten.

Schmelzpunkt 256 - 258 °C.

Das Ausgangsprodukt wird wie folgt dargestellt:

a) 5-Formyl-β-carbolin-3-carbonsäure-ethylester

5-Hydroxymethyl-β-carbolin-3-carbonsäure-ethylester (1,0 g) wird in Dichlormethan (250 ml) mit Mangandioxid (1,5 g) 16 Stunden bei Raumtemperatur (25 °C) gerührt. Nach Zugabe von weiterem Mangandioxid (0,75 g) wird das Reaktionsgemisch noch einmal 16 Stunden gerührt. Anschließend wird es nach Abfiltrieren der ungelösten Anteile eingedampft, der Rückstand wird zweimal aus Essigester umkristallisiert. Es werden so 0,5 g 5-Formyl-β-carbolin-3-carbonsäure-ethylester vom Schmelzpunkt 273 - 276 °C erhalten.

b) 5-Phenyliminomethyl-β-carbolin-3-carbonsäure-ethylester

5-Formyl-β-carbolin-3-carbonsäure-ethylester (0,153 g) wird mit Anilin (0,112 g) in Essigsäure (3 ml) unter Stickstoff eine Stunde bei 25 °C gerührt. Die ausgefallenen Kristalle werden abgesaugt. Die Ausbeute beträgt 0,111 g 5-Phenyliminomethyl-β-carbolin-3-carbonsäure-ethylester vom Schmelzpunkt 298 - 302 °C.

Beispiel 2

5-(1-Imidazolylmethyl)-4-methoxymethyl-β-carbolin-3-carbonsäure-ethylester

5-Brom-methyl-4-methoxymethyl-β-carbolin-3-carbonsäure-ethylester (0,35 g) werden mit Imidazol (0,13 g) in Dimethylsulfoxid (4 ml) drei Tage bei Raumtemperatur belassen. Der nach Zugabe von Wasser (40 ml) ausgefallene Niederschlag wird aus Ethanol umkristallisiert und an Kieselgel mit einem Gemisch aus 19 Teilen Dichlormethan und einem Teil Methanol chromatographiert. Es werden so 0,2 g 5-(1-Imidazolylmethyl)-4-methoxymethyl-β-carbolin-3-carbonsäure-ethylester vom Schmelzpunkt 220 - 222 °C erhalten.

Das Ausgangsmaterial wird wie folgt hergestellt:

a) 4-Acetoxymethylindol

4-Hydroxymethylindol (17 g) wird in Pyridin (12 ml) mit Acetanhydrid (11,9 ml) drei Stunden auf dem Dampfbad erhitzt. Nach Verdünnen mit Ether wird das Reaktionsgemisch erst mit 1-normaler Salzsäure, dann mit gesättigter Natriumbicarbonatlösung und schließlich mit Wasser ausgeschüttelt. Die etherische Lösung wird eingedampft. Es bleiben 20 g 4-Acetoxymethylindol zurück.

b) 3-(4-Acetoxymethylindol-3-yl)-4-methoxy-2-nitrobuttersäure-ethylester

4-Acetoxy-methylindol (189 g) wird in einer Mischung aus Toluol (6 l) und Essigsäure (0,7 l) gelöst. Zu dieser Lösung wird 3-Hydroxy-2-nitro-5-oxa-hexansäure-ethylester (570 ml) gegeben. Der das Gemisch enthaltende Kolben wird mittels einer Wasserstrahlpumpe evakuiert. Danach wird mit Argon der Ausgleich zum Normaldruck herbeigeführt. Evakuieren und Druckausgleich werden viermal wiederholt. Anschließend wird das Reaktionsgemisch in einer wasserfreien Argonatmosphäre zwei Stunden am Rückfluß gekocht. Nach Einengen auf 2 l wird die Lösung mit Essigsäure-ethylester verdünnt und dreimal mit je einem Liter 1-normaler Salzsäure ausgeschüttelt. Danach wird sie mit gesättigter Kochsalzlösung neutralgewaschen. Die über Natriumsulfat getrocknete Lösung wird eingedampft. Der Rückstand wird an Kieselgel mit Dichlormethan chromatographiert. Es werden 393 g der Titelverbindung in öliger Form erhalten.

c) 3-(4-Acetoxymethylindol-3-yl)-2-amino-4-methoxy-buttersäure-ethylester

3-(4-Acetoxymethylindol-3-yl)-2-nitro-4-methoxy-buttersäure-ethylester (226 g) wird in Ethanol (2,3 l) mit Raney-Nickel als Katalysator unter Wasserstoff von Normaldruck ohne Wärmezufuhr hydriert. 3 Mol Wasserstoff werden in 3 1/2 Stunden aufgenommen, wobei die Temperatur ein Maximum von 45 °C erreicht. Nach Abfiltrieren des Katalysators und Eindampfen des Rückstandes wird das Rohprodukt an Kieselgel mit einem Gemisch aus Dichlormethan (97,5 %) und Ethanol (2,5 %) chromatographiert. Es werden 130 g der Titelverbindung als nichtkristallines Diastereomerengemisch erhalten.

d) 5-Acetoxymethyl-4-methoxymethyl-1,2,3,4-tetrahydro-β-carbolin-1,3-dicarbonsäure-3-ethylester

Zu einer auf 0 °C gekühlten Lösung von Glyoxylsäure-monohydrat (2,4 g) in Wasser (30 ml) wird unter Argonschutz und Rühren eine Lösung von 3-(4-Acetoxymethylindol-3-yl)-2-amino-4-methoxy-buttersäure-ethylester (8,7 g) in Essigsäure-ethylester (40 ml) langsam getropft. Durch Zusatz von Kaliumbicarbonat (circa 1 g) wird der pH-Wert der Lösung auf 4 eingestellt. Dann wird das Gemisch 2 Stunden gerührt, wobei man es sich auf Raumtemperatur erwärmen läßt. Essigester- und Wasserphase werden getrennt, die Wasserphase wird dreimal mit Essigester ausgeschüttelt. Die vereinigten Essigesterextrakte werden ihrerseits einmal mit Wasser gewaschen, mit Natriumsulfat getrocknet und eingedampft. 9 g der Titelverbindung bleiben zurück.

e) 5-Acetoxymethyl-4-methoxymethyl-β-carbolin-3-carbonsäure-ethylester

Zu einer Lösung von 5-Acetoxymethyl-4-methoxymethyl-1,2,3,4-tetrahydro-β-carbolin-1,3-dicarbonsäure-3-ethylester (76 g) in sauerstoffreiem Dichlormethan (0,6 l) wird unter Argonschutz 90%iger Azodicarbonäure-diethylester (76 ml) ohne Wärmezufuhr unter Rühren getropft.Die Temperatur erhöht sich um etwa 10 °C. Es wird 9 Stunden am Rückfluß gekocht, danach wird das Gemisch 60 Stunden bei

Raumtemperatur belassen. Der Niederschlag wird abgesaugt, aus der eingedampften Mutterlauge kann nach Chromatographie an Kieselgel mit einem Gemisch von Dichlormethan (95%) mit Methanol (5%) weiteres Endprodukt gewonnen werden. Insgesamt werden 65 g der Titelverbindung in Form farbloser Kristalle vom Schmelzpunkt 129 - 133 °C erhalten.

f) 5-Hydroxymethyl-4-methoxymethyl-β-carbolin-3-carbonsäure-ethylester

5-Acetoxymethyl-4-methoxymethyl-β-carbolin-3-carbonsäureethylester (7,9 g) wird in einer Lösung von Natrium (1,4 g) in Ethanol (1oo ml) aufgenommen und 4 Tage bei + 4 °C belassen. Die Lösung wird eingeengt, in reichlich Essigsäure-ethylester aufgenommen, mit Wasser alkalifrei gewaschen, mit Natriumsulfat getrocknet und im Vakuum eingedampft. Der Rückstand ergibt beim Behandeln mit Ethanol 6,6 g der Titelverbindung als farblose Kristalle vom Schmelzpunkt 139 - 140 °C.

g) 5-Brom-methyl-4-methoxymethyl-β-carbolin-3-carbonsäure-ethylester

5-Hydroxymethyl-4-methoxymethyl-β-carbolin-3-carbonsäure-ethylester (1 g) wird in Dichlormethan (50 ml) gelöst. In einer trockenen Argonatmosphäre wird eine Lösung von Phosphortribromid (0,86 g) in Dichlormethan (50 ml) zugetropft. Nach 20-stündigem Rühren bei Raumtemperatur wird der ausgefallene Niederschlag abgesaugt und mit Essigsäure-ethylester gewaschen. Es werden 1,2 g der Titelverbindung, vom Schmelzpunkt 223 - 225 °C, erhalten.

Beispiel 3

4-Methoxymethyl-5-(4-morpholinylmethyl)-β-carbolin-3-carbonsäure-ethylester

5-Hydroxymethyl-4-methoxymethyl-β-carbolin-3-carbonsäure-ethylester (0,20 g) werden in Dichlormethan (5 ml) gelöst. Unter Rühren wird eine Lösung von Phosphortribromid (0,17 g) in Dichlormethan (3 ml) zugetropft. Nach dreistündigem Rühren wird das Gemisch auf + 10 °C abgekühlt und mit einer Lösung von Morpholin (1,0 ml) in Ethanol (5 ml) tropfenweise versetzt. Nach Stehen über Nacht wird das Lösungsmittel abgedampft, der Rückstand wird an Kieselgel mit einer Mischung aus Dichlormethan (19 Teile) mit Ethanol (1 Teil) chromatographiert und man erhält die Titelverbindung (0,16 g) vom Schmelzpunkt 195 - 196 °C.

Analog werden erhalten:
4-Methoxymethyl-5-(4-methyl-1-piperazinylmethyl)-β-carbolin-3-carbonsäure-ethylester,
Schmelzpunkt 249 - 252 °C
4-Methoxymethyl-5-(1-piperidinylmethyl)-β-carbolin-3-carbonsäure-ethylester
4-Methoxymethyl-5-(2,6-dimethyl-4-morpholinylmethyl)-β-carbolin-3-carbonsäure-ethylester
4-Methoxymethyl-5-diethylaminomethyl-β-carbolin-3-carbonsäure-ethylester
4-Methyl-5-(4-morpholinylmethyl)-β-carbolin-3-carbonsäureethylester Schmelzpunkt 226-227°C
4-Methoxymethyl-5-dimethylaminomethyl-β-carbolin-3-carbonsäure-ethylester
4-Methoxymethyl-5-[2-4-morpholinyl)-ethyl]-aminomethyl-β-carbolin-3-carbonsäure-ethylester
5-[N-(2-Ethoxyethyl)-aminomethyl]-4-methoxymethyl-β-carbolin-3-carbonsäure-ethylester
Schmelzpunkt 193 - 195 °C
5-[N,N-Bis(2-methoxyethyl)-aminomethyl]-4-methoxymethyl-β-carbolin-3-carbonsäure-ethylester
Schmelzpunkt 103 - 105 °C

Beispiel 4

4-Methoxymethyl-5-(4-morpholinylmethyl)-β-carbolin-3-carbonsäure

Der nach Beispiel 3 erhaltene Ethylester (0,30 g) wird in Ethanol (30 ml) mit 1-normaler Natronlauge (2,3 ml) 4 Stunden am Rückfluß gekocht. Nach dem Erkalten wird 1-normale Essigsäure (2,3 ml) zugesetzt und eingedampft. Der kristalline Eindampfrückstand wird abgesaugt und gut mit Wasser gewaschen. So werden 0,27 g der Titelverbindung erhalten.
Schmelzpunkt 253 - 255 °C.

Analog werden erhalten:
4-Methoxymethyl-5-(2,6-dimethyl-4-morpholinyl-methyl)-β-carbolin-3-carbonsäure

Beispiel 5

4-Methoxymethyl-5-(4-morpholinylmethyl)-β-carbolin-3-carbonsäure-tert.-butylester

Die nach Beispiel 4 erhaltene Säure (0,35 g) wird in tert.-Butoxy-bis-(dimethylamino)-methan (7 ml) unter Argonschutz zwei Stunden auf 120 °C erwärmt. Nach Abdampfen des tert.-Butoxy-bis-(dimethylamino)-methan wird der Rückstand in Essigsäure-ethylester aufgenommen. Die Lösung wird mit

gesättigter Kochsalzlösung ausgeschüttelt, getrocknet und eingedampft. Der Rückstand wird an Kieselgel mit einem Gemisch aus gleichen Teilen Hexan und Aceton chromatographiert. Die Ausbeute an Titelverbindung beträgt 0,2 g.

Analog werden erhalten:
4-Methoxymethyl-5-(2,6-dimethyl-4-morpholinyl-methyl)-β-carbolin-3-carbonsäure-tert.-butylester

Beispiel 6

4-Methoxymethyl-5-(4-morpholinylmethyl)-β-carbolin-3-carbonsäure-isopropylester

5-Brom-methyl-4-methoxymethyl-β-carbolin-3-carbonsäure-isopropylester (0,53 g) wird in Ethanol (10 ml) suspendiert. Die nach Zugabe von Morpholin (2,6 ml) entstehende Lösung wird bei 25 °C 20 Stunden stehengelassen. Dann wird sie mit Essigsäure-ethylester (60 ml) verdünnt und mit Wasser ausgeschüttelt, bis sie nicht mehr alkalisch reagiert. Die neutrale Lösung wird im Vakuum eingedampft, der Rückstand aus Essigsäure-ethylester umkristallisiert. Die Ausbeute beträgt 0,16 g mit dem Schmelzpunkt 214 - 216 °C.

Das Ausgangsmaterial wird folgendermaßen dargestellt:

a) 5-Hydroxymethyl-4-methoxymethyl-β-carbolin-3-carbonsäure-isopropylester

5-Hydroxymethyl-4-methoxymethyl-β-carbolin-3-carbonsäure-ethylester (7,27 g) wird in Isopropanol (1000 ml) mit Titan-tetra-isopropylat (7,1 ml) 5 Stunden am Rückfluß gekocht. Die Lösung wird eingedampft, der Rückstand in Essigsäure-ethylester gelöst. Die vollständige Lösung wird durch Zugabe von 1-normaler Salzsäure erzielt. Anschließend wird mit 1-normaler Natronlauge alkalisch gemacht. Ein dadurch entstandener Niederschlag wird abgesaugt. Das Filtrat wird eingedampft und ergibt einen Rückstand von 6,0 g der Titelverbindung.

Analog wird erhalten aus 4-Methyl-5-(4-morpholinylmethyl)-β-carbolin-3-carbonsäureethylester der 4-Methyl-5-(4-morpholinylmethyl)-β-carbolin-3-carbonsäure-isopropylester

b) 5-Brommethyl-4-methoxymethyl-β-carbolin-3-carbonsäure-isopropylester

5-Hydroxymethyl-4-methoxymethyl-β-carbolin-3-carbonsäure-isopropylester (1,0 g) wird in Dichlormethan gelöst. Eine Lösung von 0,83 g Phosphortribromid in 10 ml Dichlormethan wird zugefügt. Nach 20 Stunden wird der entstandene Niederschlag abgesaugt. Die Ausbeute beträgt 1,0 g gelbe Kristalle mit unscharfem Schmelzpunkt.

Analog werden hergestellt:
4-Methoxymethyl-5-(1-pyrrolidinylmethyl)-β-carbolin-3-carbonsäure-isopropylester,
Schmelzpunkt 173 - 174 °C.
4-Methoxymethyl-5-(4-thiomorpholinylmethyl)-β-carbolin-4-carbonsäure-isopropylester,
Schmelzpunkt 217 - 219 °C.
4-Methoxymethyl-5-(2,6-dimethyl-4-morpholinylmethyl)-β-carbolin-3-carbonsäure-isopropylester.

Beispiel 7

5-Morpholino-methyl-β-carbolin-3-carbonsäure-ethylester

Unter Stickstoff wird eine Lösung von 0,19 g 9-Acetyl-5-brommethyl-β-carbolin-3-carbonsäure-ethylester in 5 ml Ethanol mit 1 ml Morpholin versetzt und über Nacht bei Raumtemperatur gerührt. Nach Zusatz von Wasser wird das Kristallisat abgesaugt und aus Ethanol/Diethylether umkristallisiert. Man erhält 0,11 g (64 %) Schmelzpunkt 285 °C.

Das Ausgangsmaterial wird auf folgende Weise hergestellt:

Eine Suspension von 6,2 g 9-Acetyl-5-methyl-β-carbolin-3-carbonsäure-ethylester (in üblicher Weise aus 5-Methyl-β-carbolin-3-carbonsäure-ethylester durch Einwirkung von Acetanhydrid in Pyridin erhalten) in 550 ml Tetrachlorkohlenstoff wird mit 4,5 g N-Bromsuccinimid und 0,17 g Azobis(isobutyro)nitril versetzt und mit einer 500 Watt-Lampe (Nitraphot BT, Osram) 2 Stunden bestrahlt, wobei die Mischung siedet. Sie wird heiß filtriert und das Filtrat eingeengt. Das Rohprodukt wird zweimal aus Tetrachlorkohlenstoff umkristallisiert. Man erhält 5,9 g (75%) des 5-Brommethyl-derivates. Schmelzpunkt 193 °C.

Analog Beispiel 7 werden dargestellt:
5-(4-Methylpiperazinylmethyl)-β-carbolin-3-carbonsäure-ethylester, Schmelzpunkt 287 °C.
5-[N-(1-Phenylethyl)-aminomethyl]-β-carbolin-3-carbonsäure-ethylester, Schmelzpunkt 232 °C.

Analog Beispiel 7, jedoch in Dimethylsulfoxid als Lösungsmittel, wird mit Imidazol erhalten:
9-Acetyl-5-(1-imidazolylmethyl)-β-carbolin-3-carbonsäure-ethylester, Schmelzpunkt 195 °C.

Beispiel 8

5-(1-Imidazolylmethyl)-β-carbolin-3-carbonsäure-ethylester

Eine Suspension von 0,11 g 9-Acetyl-5-(1-imidazolylmethyl)-β-carbolin-3-carbonsäure-ethylester in 5 ml Ethanol wird mit 10 mg $K_2CO_3$ versetzt und zwei Stunden unter Rückfluß erhitzt. Nach Filtration wird die Lösung im Vakuum eingeengt, der Rückstand mit Wasser versetzt, abgesaugt und aus Wasser unmristallisiert. Ausbeute 70 mg (71%).
Schmelzpunkt 248 °C.

Beispiel 9

5-(2-Aminoethyl)-4-methyl-β-carbolin-3-carbonsäure-ethylester

5-(2-Nitrovinyl)-4-methyl-β-carbolin-3-carbonsäure-ethylester (1 g) wird in Ethanol (20 ml) gelöst und in einer Wasserstoffatmosphäre unter Rühren zu einer Suspension von 10% iger Palladiumkohle (0,25 g) in Ethanol (50 ml) und Schwefelsäure (0,1 ml) langsam getropft. Zum Schluß wird noch eine halbe Stunde weitergerührt. Anschließend wird der Katalysator abgesaugt und das Filtrat eingedampft. Nach üblicher Aufarbeitung werden 0,6 g der Titelverbindung erhalten.

Die Ausgangsverbindung wird wie folgt dargestellt:

a) 5-Formyl-4-methyl-β-carbolin-3-carbonsäure-ethylester

Die Synthese erfolgt nach der im Beispiel 1 beschriebenen Methode aus 5-Hydroxymethyl-4-methyl-β-carbolin-3-carbonsäure-ethylester, der wiederum nach Beispiel 2 aus 2-Nitro-3-hydroxybuttersäure-ethylester in 5 Stufen dargestellt wird.

b) 5-(2-Nitrovinyl)-4-methyl-β-carbolin-3-carbonsäure-ethylester

Eine Lösung von 5-Formyl-4-methyl-β-carbolin-3-carbonsäure-ethylester (10 g), Nitromethan (2 ml) und Methylaminhydrochlorid (0,2 g) in Ethanol (50 ml) wird mit Natriumcarbonat (0,3 g) versetzt und unter gelegentlichem Umschütteln 5 Tage bei 20 °C belassen. Anschließend wird bis zur beginnenden Kristallisation eingedampft. Aus der mit Eiswasser gekühlten Lösung kristallisieren 5 g der Titelverbindung.

Beispiel 10

5-(2-Aminopropyl)-4-methyl-β-carbolin-3-carbonsäure-ethylester

Die Herstellung erfolgt analog Beispiel 9 durch Kondensation von 5-Formyl-4-methyl-β-carbolin-3-carbonsäure-ethylester mit Nitroethan und Hydrierung des entstandenen 4-Methyl-5-(2-nitropropenyl)-β-carbolin-3-carbonsäure-ethylesters

Beispiel 11

4-Methoxymethyl-5-morpholinomethyl-3-(3-ethyl-1,2,4-oxadiazol-5-yl)β-carbolin

Eine Lösung von 0,36 g 4-Methoxymethyl-5-morpholinomethyl-β-carbolin-3-carbonsäure (dargestellt nach Beispiel 4) in 10 ml absolutem Dimethylformamid wird mit 0,2 g Carbonyl-diimidazol versetzt und 30 Minuten bei 60 °C gerührt. Anschließend wird 0,4 g Propioamidoxim in 2 ml DMF zugesetzt und das Reaktionsgemisch 3 Stunden auf 100 °C erhitzt. Nach Abdestillieren des Lösungsmittel im Vakuum wird der Rückstand mit 20 ml Xylol versetzt und 3 Stunden unter Rückfluß am Wasserabscheider gekocht, das Reaktionsgemisch wird heiß filtriert, das Filtrat eingeengt und an Kieselgel chromatographiert. Man erhält 0,25 g (60 %) Oxadiazol-derivat. Schmelzpunkt 161-163°C.

4-Methyl-5-(4-morpholinylmethyl)-3-(3-ethyl-1, 2, 4-oxadiazol-5-yl)-β-carbolin

4-Methyl-5-(4-morpholinylmethyl)-β-carbolin-3-carbonsäure-hydrochlorid (0,46 g) wird in Dimethylformamid (35 ml) suspendiert und mit Carbonyldiimidazol (0,46g) versetzt. Der klaren Lösung wird nach 24 Stunden bei Raumtemperatur Propionamidoxim (0,5 g) zugefügt. Nach 48 Stunden bei Raumtemperatur wird das Dimethylformamid im Vakuum abdestilliert. Das zurückbleibende Öl wird in Xylol (50 ml) 3 Stunden am Wasserabscheider unter Rückfluß gekocht, anschließend wird das Xylol dekantiert und eingedampft. Der Rückstand wird aus Essigester umkristallisiert.

Ausbeute 0,3 g, Schmelzpunkt 189-190°C. Das Ausgangsmaterial wird auf folgende Weise hergestellt:

Eine Lösung von 4-Methyl-5-(4-morpholinylmethyl-β-carbolin-3-carbonsäure-ethylester (0,5 g) in Ethanol (40ml) und 1-normaler Natronlauge (4,3 ml) wird 4 Stunden am Rückfluß gekocht. Nach dem Erkalten wird 1-normale Salzsäure (8,7ml) zugesetzt. Die klarfiltrierte Lösung wird 2 Tage bei +4°C belassen, anschließend wird der in dieser Zeit ausgefallene Niederschlag abgesaugt. Die Ausbeute beträgt 0,47 g, Schmelzpunkt 270-273°C.

## Patentansprüche

1. 5-Aminoalkyl-β-carbolinderivate der allgemeinen Formel I

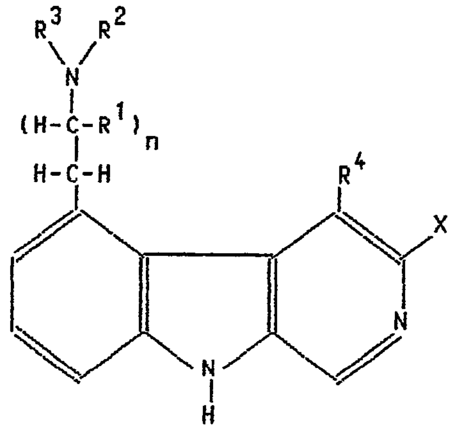

(I),

worin
n= 0 oder 1 ist,
$R^1$ Wasserstoff oder $C_{1-4}$-Alkyl bedeutet,
$R^2$ und $R^3$ jeweils Wasserstoff, Alkanoyl mit bis zu 4 Kohlenstoffatomen, Phenyl oder $C_{1-4}$-Alkyl, das substituiert sein kann mit Hydroxy, $C_{1-4}$-Alkoxy, Merkapto, $C_{1-4}$-Alkylthio, Phenyl, einer gegebenenfalls mit $C_{1-4}$-Alkyl substituierten Aminogruppe oder einem gegebenenfalls mit 1 oder 2 $C_{1-4}$-Alkylgruppen substituierten Morpholin-, Piperidin-, Thiomorpholin-, Piperazin-, Pyrrolidin- oder 4-($C_{1-4}$-Alkyl)piperazin-Rest, darstellen oder gemeinsam mit dem Stickstoffatom einen gegebenenfalls mit 1 oder 2 $C_{1-4}$-Alkylgruppen substituierten Morpholin-, Thiomorpholin-, Piperidin-, Pyrrolidin-, Piperazin- oder 4-($C_{1-4}$-Alkyl)-piperazin Rest bedeuten, oder einen Imidazol-, Pyrazol- oder Pyrrol-Rest bedeuten,
$R^4$ Wasserstoff, $C_{1-4}$-Alkyl oder $C_{1-4}$-Alkoxyalkyl bedeutet und
X einen Oxadiazolylrest der Formel

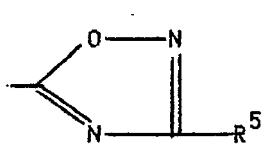

oder

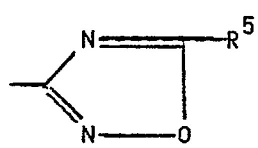

mit $R^5$ in der Bedeutung von H, $C_{1-4}$-Alkyl oder $C_{3-7}$-Cycloalkyl darstellt oder
eine $COOR^6$-Gruppe mit $R^6$ in der Bedeutung von H oder $C_{1-6}$-Alkyl ist oder
eine CO-$NR^7$ $R^8$ -Gruppe, wobei $R^7$ und $R^8$ jeweils $C_{1-3}$-Alkyl oder gemeinsam mit dem Stickstoffatom eine Morpholin-, Piperidin-, Thiomorpholin-, Piperazin-, Pyrrolidin- oder 4-($C_{1-4}$-Alkyl)-piperazin-Rest bedeuten der mit 1 oder 2 $C_{1-4}$-Alkylresten substituiert sein kann.

2. 5-Phenylaminomethyl-β-carbolin-3-carbonsäure-ethylester
5-Diethylaminomethyl-4-methoxymethyl-β-carbolin-3-carbonsäure-ethylester
5-[2-(4-morpholinyl)-ethyl-aminomethyl]-4-methoxymethyl-β-carbolin-3-carbonsäure-ethylester
5-[N-(2-Ethoxyethyl)-aminomethyl]-4-methoxymethyl-β-carbolin-3-carbonsäure-ethylester
5-Dimethylaminomethyl-4-methoxymethyl-β-carbolin-3-carbonsäure-ethylester
5-[N,N-Bis(2-methoxyethyl)-aminomethyl]-4-methoxymethyl-β-carbolin-3-carbonsäure-ethylester
5-[N-(1-Phenylethyl)-aminomethyl]-β-carbolin-3-carbonsäure-ethylester.

3. 5-(1-Imidazolylmethyl)-4-methoxymethyl-β-carbolin-3-carbonsuäre-ethylester
5-(1-Imidazolylmethyl)-β-carbolin-3-carbonsäure-ethylester)

4. 5-(4-morpholinylmethyl)-4-methoxymethyl-β-carbolin-3-carbonsäure-ethylester
5-(4-methyl-1-piperazinylmethyl)-4-methoxymethyl-β-carbolin-3-carbonsuäre-ethylester
5-(1-piperidinylmethyl)-4-methoxymethyl-β-carbolin-3-carbonsäure-ethylester
5-(2,6-dimethyl-4-morpholinylmethyl)-4-methoxymethyl-β-carbolin-3-carbonsäure-ethylester
5-Morpholinylmethyl-β-carbolin-3-carbonsäure-ethylester
5-(4-Methylpiperazinylmethyl)-β-carbolin-3-carbonsäure-ethylester.

5. 5-(2-Aminoethyl)-4-methyl-β-carbolin-3-carbonsäure-ethylester
5-(2-Aminopropyl)-4-methyl-β-carbolin-3-carbonsäure-ethylester.
6. 5-Morpholinomethyl-4-methoxymethyl-3-(3-ethyl-1,2,4-oxadiazol-5-yl)-β-carbolin.
7. 5-(4-morpholinylmethyl)-4-methoxymethyl-β-carbolin-3-carbonsäure
5-(2,6-dimethyl-4-morpholinylmethyl)-4-methoxymethyl-β-carbolin-3-carbonsäure.
8. 5-(4-morpholinylmethyl)-4-methoxymethyl-β-carbolin-3-carbonsäure-tert.-butylester
5-(2,6-dimethyl-4-morpholinylmethyl)-4-methoxymethyl-β-carbolilin-3-carbonsäure-tert.-butylester
5-(4-morpholinylmethyl)-4-methoxymethyl-β-carbolin-3-carbonsäure-isopropylester
5-(1-pyrrolidinylmethyl)-4-methoxymethyl-β-carbolin-3-carbonsäure-isopropylester
5-(4-thiomorpholinylmethyl)-4-methoxymethyl-β-carbolin-3-carbonsäure-isopropylester.
5-(2,6-dimethyl-4-morpholinylmethyl)-4-methoxymethyl-β-carbolin-3-carbonsäure-isopropylester
9.) Verfahren zur Herstellung der Verbindungen der allgemeinen Formel I, dadurch gekennzeichnet, daß man
a) eine Verbindung der allgemeinen Formel II

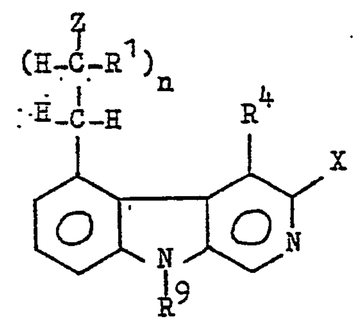

(II),

worin
$R^1$, $R^4$, X und n die oben angegebene Bedeutung haben,
Z Halogen oder Hydroxy ist und
$R^9$ Wasserstoff oder eine Schutzgruppe bedeutet
mit einer Verbindung $HNR^2R^3$, worin $R^2$ und $R^3$ die oben angegebene Bedeutung haben, umsetzt und anschließend gegebenenfalls die Schutzgruppe abspaltet,
b) eine Verbindung der allgemeinen Formel III

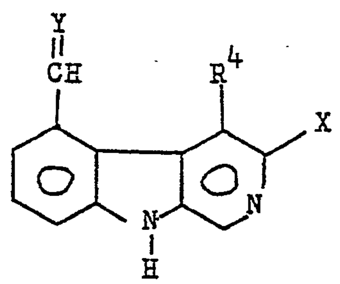

(III),

worin
$R^4$ und X die oben genannte Bedeutung haben und
Y eine $O_2N$-C-$R^1$- oder $R^2$N-Gruppe ist, worin $R^1$ die oben genannte Bedeutung hat und $R^2$ gegebenenfalls substituiertes niederes Alkyl oder Aryl darstellt,
hydriert zu einer Verbindung der allgemeinen Formel I mit $R^3$ in der Bedeutung von Wasserstoff und anschließend gegebenenfalls die nach Verfahren a) oder b) erhaltenen Verbindungen umestert oder die Ester verseift und gewünschtenfalls die so erhaltene Carbonsäure
α) amidiert
β) mit einer Verbindung

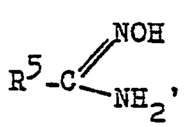

worin $R^5$ die oben genannte Bedeutung hat, umsetzt zu einer Verbindung der allgemeinen Formel I, in der X für den Rest

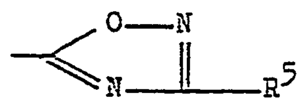

mit $R^5$ in der oben genannten Bedeutung steht,
c) eine Verbindung der allgemeinen Formel IV

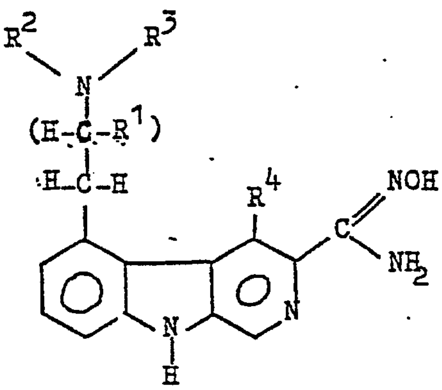

(IV),

worin
$R^1$, $R^2$, $R^3$, $R^4$ und n die oben genannte Bedeutung haben mit einem Carbonsäureanhydrid $(R^5CO)_2O$, worin $R^5$ die oben genannte Bedeutung hat, umsetzt zu einer Verbindung der allgemeinen Formel I, in der X für den Rest

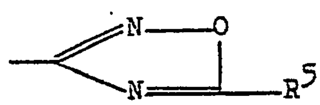

mit $R^5$ in der oben genannten Bedeutung steht.
10.)Verwendung der Verbindungen gemäß Anspruch 1 bis 8 als Arzneimittel.

## Claims

1. 5-Aminoalkyl-β-carboline derivatives of the general formula I

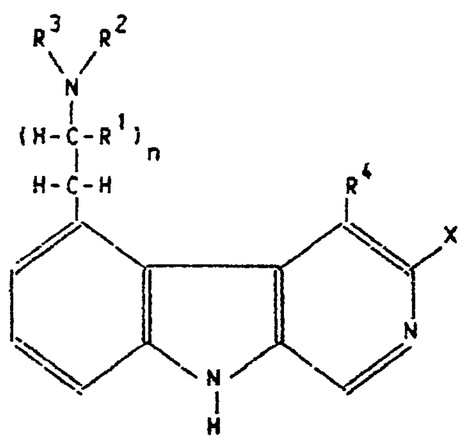

(I)

wherein
n = 0 or 1,
$R^1$ represents hydrogen or $C_{1-4}$-alkyl,
$R^2$ and $R^3$ are each hydrogen, alkanoyl having up to 4 carbon atoms, phenyl or $C_{1-4}$-alkyl that may be substituted by hydroxy, $C_{1-4}$-alkoxy, mercapto, $C_{1-4}$-alkylthio, phenyl, by an amino group optionally substituted by $C_{1-4}$-alkyl, or by a morpholine, piperidine, thiomorpholine, piperazine, pyrrolidine or 4-($C_{1-4}$-alkyl)-piperazine radical each optionally substituted by 1 or 2 $C_{1-4}$-alkyl groups, or

$R_2$ and $R_3$ together with the nitrogen atom represent a morpholine, thiomorpholine, piperidine, pyrrolidine, piperazine or 4-($C_{1-4}$-alkyl)-piperazine radical each optionally substituted by 1 or 2 $C_{1-4}$-alkyl groups, or represent an imidazole, pyrazole or pyrrole radical,
$R^4$ represents hydrogen, $C_{1-4}$-alkyl or $C_{1-4}$-alkoxyalkyl and
X represents an oxadiazolyl radical of the formula

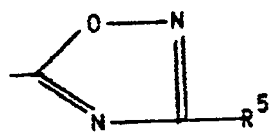

or

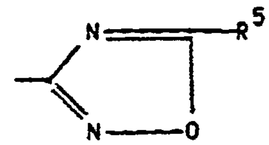

in which $R^5$ represents H, $C_{1-4}$-alkyl or $C_{3-7}$-cycloalkyl, or
a $COOR^6$ group in which $R^6$ represents H or $C_{1-6}$-alkyl, or
a $CO-NR^7R^8$ group in which $R^7$ and $R^8$ each represent $C_{1-3}$-alkyl or together with the nitrogen atom a morpholine, piperidine, thiomorpholine, piperazine, pyrrolidine or 4-($C_{1-4}$-alkyl)-piperazine radical, which may be substituted by 1 or 2 $C_{1-4}$-alkyl radicals.

2. 5-Phenylaminomethyl-β-carboline-3-carboxylic acid ethyl ester
5-diethylaminomethyl-4-methoxymethyl-β-carboline-3-carboxylic acid ethyl ester
5-[2-(4-morpholinyl)-ethylaminomethyl]-4-methoxymethyl-β-carboline-3-carboxylic acid ethyl ester
5-[N-(2-ethoxyethyl)-aminomethyl]-4-methoxymethyl-β-carboline-3-carboxylic acid ethyl ester
5-dimethylaminomethyl-4-methoxymethyl-β-carboline-3-carboxylic acid ethyl ester
5-[N,N-bis(2-methoxyethyl)-aminomethyl]-4-methoxymethyl-β-carboline-3-carboxylic acid ethyl ester
5-[N-(1-phenylethyl)-aminomethyl]-β-carboline-3-carboxylic acid ethyl ester.

3. 5-(1-Imidazolylmethyl)-4-methoxymethyl-β-carboline-3-carboxylic acid ethyl ester
5-(1-imidazolylmethyl)-β-carboline-3-carboxylic acid ethyl ester.

4. 5-(4-Morpholinylmethyl)-4-methoxymethyl-β-carboline-3-carboxylic acid ethyl ester
5-(4-methyl-1-piperazinylmethyl)-4-methoxymethyl-β-carboline-3-carboxylic acid ethyl ester
5-(1-piperidinylmethyl)-4-methoxymethyl-β-carboline-3-carboxylic acid ethyl ester
5-(2,6-dimethyl-4-morpholinylmethyl)-4-methoxymethyl-β-carboline-3-carboxylic acid ethyl ester
5-morpholinylmethyl-β-carboline-3-carboxylic acid ethyl ester
5-[4-methylpiperazinylmethyl)-β-carboline-3-carboxylic acid ethyl ester.

5. 5-(2-Aminoethyl)-4-methyl-β-carboline-3-carboxylic acid ethyl ester
5-(2-aminopropyl)-4-methyl-β-carboline-3-carboxylic acid ethyl ester.

6. 5-Morpholinomethyl-4-methoxymethyl-3-(3-ethyl-1,2,4-oxadiazol-5-yl)-β-carboline.

7. 5-(4-Morpholinylmethyl)-4-methoxymethyl-β-carboline-3-carboxylic acid
5-(2,6-dimethyl-4-morpholinylmethyl)-4-methoxymethyl-β-carboline-3-carboxylic acid.

8. 5-(4-Morpholinylmethyl)-4-methoxymethyl-β-carboline-3-carboxylic acid tert.-butyl ester
5-(2,6-dimethyl-4-morpholinylmethyl)-4-methoxymethyl-β-carboline-3-carboxylic acid tert.-butyl ester
5-(4-morpholinylmethyl)-4-methoxymethyl-β-carboline-3-carboxylic acid isopropyl ester
5-(1-pyrrolidinylmethyl)-4-methoxymethyl-β-carboline-3-carboxylic acid isopropyl ester
5-(4-thiomorpholinylmethyl)-4-methoxymethyl-β-carboline-3-carboxylic acid isopropyl ester
5-(2,6-dimethyl-4-morpholinylmethyl)-4-methoxymethyl-β-carboline-3-carboxylic acid isopropyl ester.

9. A process for the preparation of compounds of the general formula I, characterised in that
a) a compound of the general formula II

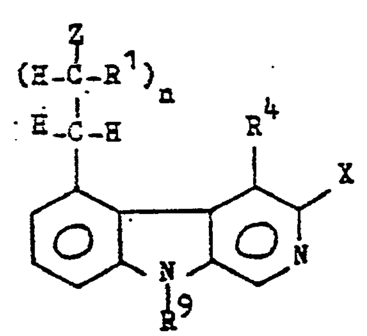

(II)

in which
$R^1$, $R^4$, X and n are as defined hereinbefore,
Z is halogen or hydroxy and
$R^9$ represents hydrogen or a protecting group, is reacted with a compound $HNR^2R^3$ in which $R^2$ and $R^3$ are as defined hereinbefore, and then, where applicable, the protecting group is removed,

b) a compound of the general formula III

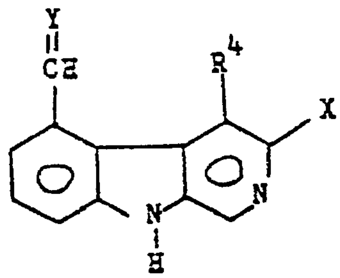

(III)

in which
$R^4$ and X are as defined hereinbefore and
Y represents an $O_2N$-C-$R^1$- or $R^2N$- group in which $R^1$ is as defined hereinbefore and $R^2$ is optionally substituted lower alkyl or aryl, is hydrogenated to a compound of the general formula I in which $R^3$ represents hydrogen, and then, optionally, the compounds obtained according to process a) or b) are transesterified or the esters are hydrolysed and, if desired, the carboxylic acid so obtained is
α) amidated,
β) reacted with a compound

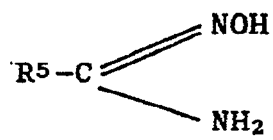

in which $R^5$
is as defined hereinbefore, to form a compound of the general formula I in which X represents the radical

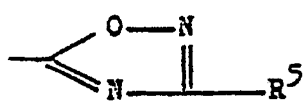

wherein $R^5$ is as defined hereinbefore,
c) a compound of the general formula IV

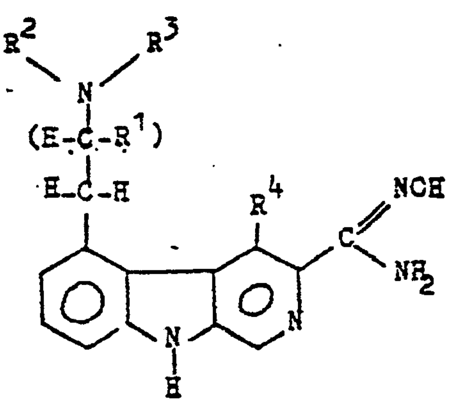

(IV),

in which
$R^1$, $R^2$, $R^3$, $R^4$ and n are as defined hereinbefore, is reacted with a carboxylic acid anhydride $(R^5CO)_2O$ wherein $R^5$ is as defined hereinbefore, to form a compound of the general formula I in which X represents the radical

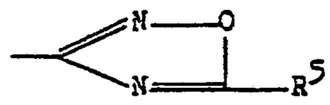

wherein $R^5$ is as defined hereinbefore.

10. The use of compounds according to claims 1 to 8 as medicaments.

**Revendications**

1. Aminoalkyl-5 β-carbolines répondant à la formule générale

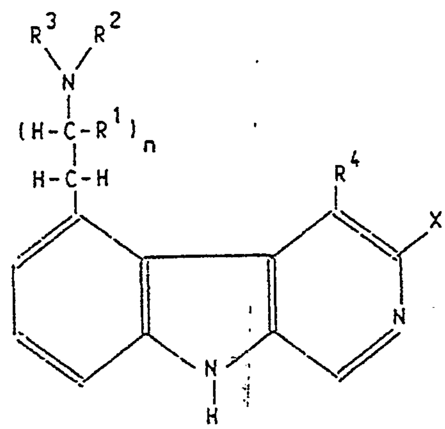

(I)

dans laquelle
n représente un nombre égal à 0 ou à 1,
$R^1$ représente l'hydrogène ou un alkyle en $C_1$–$C_4$,
$R^2$ et $R^3$ représentent chacun l'hydrogène, un alcanoyle contenant au plus 4 atomes de carbone, un phényle ou un alkyle en $C_1$–$C_4$, lequel peut porter un hydroxy, un alcoxy en $C_1$–$C_4$, un mercapto, un alkylthio en $C_1$–$C_4$, un phényle, un radical amino éventuellement porteur d'un alkyle en $C_1$–$C_4$ ou un radical de morpholine, de pipéridine, de thiomorpholine, de pipérazine, de pyrrolidine ou d'(alkyl-$C_1$–$C_4$)-4 pipérazine éventuellement porteur d'un ou de deux alkyles en $C_1$–$C_4$, ou forment ensemble, et avec l'atome d'azote, un radical de morpholine, de thiomorpholine, de pipéridine, de pyrrolidine, de pipérazine ou d'(alkyl-$C_1$–$C_4$)-4 pipérazine éventuellement porteur d'un ou de deux alkyles en $C_1$–$C_4$, ou un radical d'imidazole, de pyrazole ou de pyrrole,
$R^4$ représente l'hydrogène, un alkyle en $C_1$–$C_4$ ou un alcoxyalkyle en $C_1$–$C_4$ et
X représente:
– un radical oxadiazolyle répondant à l'une des formules:

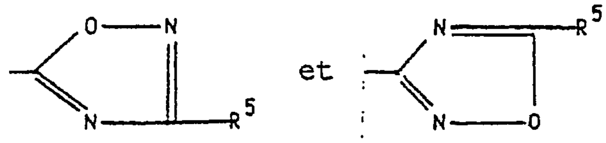

dans lesquelles $R^5$ représente H, un alkyle en $C_1$–$C_4$ ou un cycloalkyle en $C_3$–$C_7$, ou
– un radical –$COOR^6$ dans lequel $R^6$ représente H ou un alkyle en $C_1$–$C_6$, ou
– un radical –CO-$NR^7R^8$ dans lequel $R^7$ et $R^8$ représentent chacun un alkyle en $C_{1-3}$ ou forment ensemble et avec l'atome d' azote un radical de morpholine, de piperidine, de thiomorpholine, de pipérazine, de pyrrolidine ou d'(alkyl-$C_1$–$C_4$)-4 pipérazine qui peut porter un ou deux alkyles en $C_1$–$C_4$.

2. Ester éthylique de l'acide phénylaminométhyl-5-β-carboline-carboxylique-3,
ester éthylique de l'acide diéthylaminométhyl-5 méthoxyméthyl-4-β-carboline-carboxylique-3,
ester éthylique de l'acide [(morpholinyl-4)-2 éthyl-aminométhyl]-5 méthoxyméthyl-4 β-carboline-carboxylique-3,
ester éthylique de l'acide [N-(éthoxy-2 éthyl)-aminométhyl]-5 méthoxyméthyl-4 β-carboline-carboxylique-3,
ester éthylique de l'acide diméthylaminométhyl-5 méthoxyméthyl-4 β-carboline-carboxylique-3,
ester éthylique de l'acide [N,N-bis-(méthoxy-2 éthyl)-aminométhyl]-5 méthoxyméthyl-4 β-carboline-carboxylique-3,
ester éthylique de l'acide [N-(phényl-l éthyl)-aminométhyl]-5 β-carboline-carboxylique-3.

3. Ester éthylique de l'acide (imidazolyl-1 méthyl)-5 méthoxyméthyl-4 β-carboline-carboxylique-3, ester éthylique de l'acide (imidazolyl-1 méthyl)-5 β-carboline-carboxylique-3.

4. Ester éthylique de l'acide (morpholinyl-4 méthyl)-5 méthoxyméthyl-4 β-carboline-carboxylique-3, ester éthylique de l'acide (méthyl-4 pipérazinyl-1 méthyl)-5 méthoxyméthyl-4 β-carboline-carboxylique-3, ester éthylique de l'acide (pipéridinyl-1 méthyl)-5 méthoxyméthyl-4 β-carboline-carboxylique-3, ester éthylique de l'acide (diméthyl-2,6 morpholinyl-4 méthyl)-5 méthoxyméthyl-4 β-carboline-carboxylique-3, ester éthylique de l'acide morpholinylméthyl-5 β-carboline-carboxylique-3, ester éthylique de l'acide (méthyl-4 pipérazinylméthyl)-5 β-carboline-carboxylique-3.

5. Ester éthylique de l'acide (amino-2 éthyl)-5 méthyl-4 β-carboline-carboxylique-3, ester éthylique de l'acide (amino-2 propyl)-5 méthyl-4 β-carboline-carboxylique-3.

6. Morpholinométhyl-5 méthoxyméthyl-4 (éthyl-3 oxadiazole-1,2,4 yl-5)-3 β-carboline.

7. Acide (morpholinyl-4 méthyl)-5 méthoxyméthyl-4 β-carboline-carboxylique-3, acide (diméthyl-2,6 morpholinyl-4 méthyl)-5 méthoxyméthyl-4 β-carboline-carboxylique-3.

8. Ester tert-butylique de l'acide (morpholinyl-4 méthyl)-5 méthoxymethyl-4 β-carboline-carboxylique-3, ester tert-butylique de l'acide (diméthyl-2,6 morpholinyl-4 méthyl)-5 méthoxyméthyl-4 β-carboline-carboxylique-3, ester isopropylique de l'acide (morpholinyl-4 méthyl)-5 méthoxyméthyl-4 β-carboline-carboxylique-3, ester isopropylique de l'acide (pyrrolidinyl-1 méthyl)-5 méthoxyméthyl-4 β-carboline-carboxylique-3, ester isopropylique de l'acide (thiomorpholinyl-4 méthyl)-5 méthoxyméthyl-4 β-carboline-carboxylique-3, ester isopropylique de l'acide (diméthyl-2–6 morpholinyl-4 méthyl)-5 méthoxyméthyl-4 β-carboline-carboxylique-3.

9. Procédé pour préparer les composés de formule générale I, procédé caractérisé en ce que:

a) on fait réagir un composé répondant à la formule générale II.

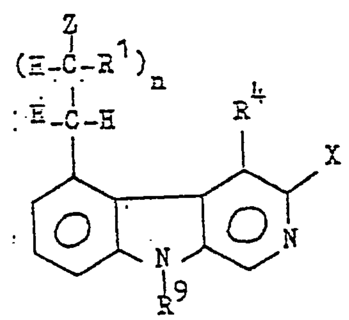

(II)

dans laquelle
$R^1$, $R^4$, X et n ont les significations qui leur ont été données ci-dessus,
Z représente un halogène ou un radical hydroxy et
$R^9$ représente l'hydrogène ou un radical protecteur, avec un composé $HNR^2R^3$ dans lequel $R^2$ et $R^3$ ont les significations qui leur ont été données ci-dessus, puis on élimine éventuellement le radical protecteur,

b) on hydrogène un composé répondant à la formule générale III

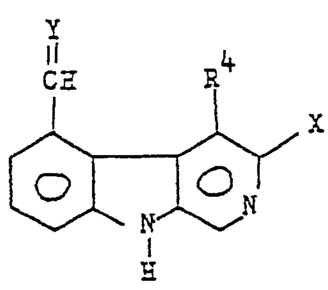

III

dans laquelle
$R^4$ et X ont les significations qui leur ont été données ci-dessus et
Y représente un radical $O_2N$-C-$R^1$ ou un radical $R^2N$ dans lesquels $R^1$ a la signification indiquée ci-dessus et $R^2$ représente un radical alkyle ou aryle éventuellement substitué, hydrogénation qui conduit à un composé de formule générale I dans lequel $R^3$ représente l'hydrogène, après quoi, le cas

échéant, on transestérifie les composés obtenus par la méthode a) ou b), ou on saponifie les esters et, si on le désire, on effectue sur l'acide carboxylique ainsi obtenu l'une des réactions suivantes:
α) une amidation
β) une réaction avec un composé

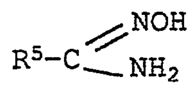

dans lequel $R^5$ a la signification indiquée ci-dessus, de manière à obtenir un composé de formule générale I dans lequel X représente un radical:

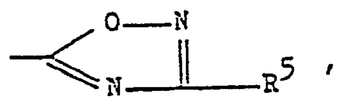

le symbole $R^5$ ayant la signification indiquée plus haut,
c) on fait réagir un composé répondant à la formule générale IV

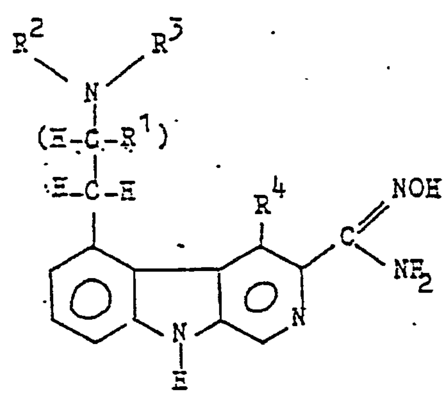

dans laquelle
$R^1$, $R^2$, $R^3$, $R^4$ et n ont les significations qui leur ont été données ci-dessus, avec un anhydride d'acide carboxylique $(R^5CO)_2O$ dans lequel $R^5$ a la signification indiquée ci-dessus, de manière à obtenir un composé de formule générale I dans lequel X désigne un radical:

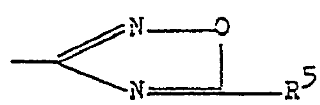

(où $R^5$ a la signification indiquée ci-dessus).
10. Application des composés selon l'une des revendications 1–8 comme médicaments.